# EUROPEAN PATENT SPECIFICATION

(11) **EP 1 390 112 B1**
(45) Date of publication and mention of the grant of the patent: **30.11.2005**
(21) Application number: 02737040.2
(22) Date of filing: 21.05.2002
(51) Int. Cl.: B01D 9/00, F28D 20/02

(54) **TRIGGER MECHANISM FOR INITIATING A PHASE CHANGE IN A VARIABLE LIQUID ELEMENT**
AUSLÖSEVORRICHTUNG ZUR INITIIERUNG EINES PHASENWECHSELS IN EINEM VERÄNDERLICHEN FLÜSSIGKEITSELEMENT
MECANISME DECLENCHEUR POUR AMORCER UN CHANGEMENT DE PHASE DANS UN ELEMENT LIQUIDE VARIABLE

(30) Priority: 31.05.2001 US 294843 P
(43) Date of publication of application: 25.02.2004
(73) Proprietor: THE PROCTER & GAMBLE COMPANY, Cincinnati, Ohio 45202 (US)
(72) Inventor: FURUMICHI, Hiroshi, Kako-gun, Hyogo 675-1113 (JP); NAGASAWA, Shunichi, Sanda-shi, Hyogo 669-1324 (JP)
(74) Representative: Clemo, Nicholas Graham
(86) International application number: PCT/US2002/016047
(87) International publication number: WO 2002/096532

(56) References cited:
- EP-A- 0 498 428
- EP-A- 0 999 149
- FR-A- 736 030
- FR-A- 2 348 121
- US-A- 3 940 905
- US-A- 5 662 096
- US-A- 5 792 213
- US-A- 5 819 923

## Description

### FIELD OF THE INVENTION

The present invention relates to a trigger mechanism for initiating a phase change in a variable liquid element by an activating material. The trigger mechanism also relates to a variable liquid element enclosed in a container with at least one perforation in the container, an activating material, and a movable barrier means for bringing the activation material into contact with the perforation.

### BACKGROUND OF THE INVENTION

A trigger mechanism for initiating a phase change is known from EP 0 498 428.

It is generally said that there are three principle states of matter - gas, liquid, and solid. While the transition between the three states is generally unhindered, occasionally external forces initiate the transition. For example, some liquids, called supercooled solutions, require a seed crystal to initiate the transition from liquid to a crystalline solid.

The use of supercooled solutions for providing heat is well known in the art. Certain salts once melted into a solution will remain liquid at a temperature below the melting point of the salt. On solidification, these supercooled solutions give up heat. Typically, water/salt combinations are used to make such supercooled solutions. In order to initiate heat dissemination, the supercooled solution must begin to solidify. Several techniques are known to initiate solidification.

One method for initiating solidification involves introducing a crystal or another type of nucleating particle into the supercooled solution. Previously suggested forms for this type of initiation include the use of flexible metal strips, usually with holes or slits that are placed into the supercooled solution. Examples are illustrated in US 4,077,390; US 4,379,448; US 4,460,546; US 4,572,158; US 5,736,110; and US 5,915,461. The use of a helically-coiled, resilient metallic filament that initiates crystallization when rubbing action is sufficient to initiate solidification is discussed in US 4,829,980. These initiation techniques are generally confined to a specific location within the solution for easier activation by the user.

Other forms for initiation of solidification of a supercooled solution include a puncturing device that admits air into the supercooled solution and thereby initiates crystallization, see US 5,305,733 and US 5,662,096. Coating the nucleating particles and placing the coated nucleating particles directly into the supercooled solution is discussed in US 6,103,139.

Each of these forms for initiation contains drawbacks that are solved by the present invention. The inclusion of the nucleating particle or device within the supercooled solution results in premature initiation of the solidification of a supercooled solution, which results in unreliability. Puncture methods also tend to be unreliable since the puncturing forces can be accidentally activated during transportation or storage of the product. Puncture methods are also undesirable when the supercooled solution is being used to heat another product where contamination of the product is undesirable. Thus there remains a need for a safe and reliable mechanism for initiating phase changes for liquids through activating materials such as nucleating particles.

### SUMMARY OF THE INVENTION

The present invention relates to a trigger mechanism for initiating a phase change in a variable liquid element by an activating material. The trigger mechanism also relates to a variable liquid element enclosed in a container having at least one wall, an activation material located external to the container of the variable liquid element, at least one perforation in the container wall, and a movable barrier means for bringing the activation material into contact with the perforation. The movable barrier means seals the perforation and isolates the perforation and the activation material so that the variable liquid element is not activated until the movable barrier means is initiated by the user.

These and other features, aspects, advantages, and variations of the present invention will become evident to those skilled in the art from a reading of the present disclosure.

### BRIEF DESCRIPTION OF THE DRAWINGS

While the specification concludes with claims particularly pointing out and distinctly claiming the invention, it is believed that the invention will be better understood from the following description of the accompanying figures, not necessarily drawn to scale, in which:
Fig. 1 is a top view of one preferred embodiment of the trigger mechanism having a one-piece configuration in an unfolded orientation;
Fig. 2 is a side view of the trigger mechanism shown in Fig. 1 taken along section line 2-2 on a container;
Fig. 3 is a top view of the trigger mechanism shown in Fig. 1 on a container;
Fig. 4 is a top view of another preferred embodiment of the one-piece trigger mechanism having a one-piece configuration in an unfolded orientation;
Fig. 5 is a side view of the trigger mechanism shown in Fig. 4 taken along section line 5-5 on a container;
Fig. 6 is a top view of the trigger mechanism shown in Fig. 4 on a container;
Fig. 7 is a partial view of a container with a preferred embodiment of a two-piece trigger mechanism attached to a package with a perforation in a wall of the package;
Fig. 8 is a view of the base tape of the trigger mechanism shown in Fig. 6 after it has been folded to bring the activation material into contact with the perforation in the wall of the container;
Fig. 9 is a top view of a preferred embodiment of a two-piece trigger mechanism in an unfolded orientation;
Fig. 10 is a view of a preferred embodiment of a two-piece trigger mechanism attached to a container with a perforation in a wall of the package; and
Fig. 11 is another preferred embodiment of the trigger mechanism shown in Fig. 10 in a one-piece configuration.

### DETAILED DESCRIPTION OF THE INVENTION

All percentages, ratios and proportions herein are by weight of the final composition, unless otherwise specified. All temperatures are in degrees Celsius (°C) unless otherwise specified. Citation of any reference is not an admission regarding any determination as to its availability as prior art to the claimed invention.

### VARIABLE LIQUID ELEMENT

A variable liquid element as used herein means any material that changes color, temperature, phase and/or another physical property when activated by a seed crystal or other nucleating particle. An example is a supercooled salt solution that can be initiated with a nucleation particle. Sodium formate (HCOONa), potassium formate (HCOOK), sodium acetate (CH₃ COONa), magnesium acetate [(CH₃COO)₂Mg], potassium nitrate (KNO₃), calcium nitrate [Ca(NO₃)₂], sodium thiosulfate (Na₂S₂O₃), dipotassium hydrogen phosphate (K₂HPO₄), and urea [(NH₂)₂CO], or any other similar salts and mixtures thereof are examples of suitable salts. The variable liquid element may also include additives for controlling the rate of reaction such as those discussed in US 4,331,556. US 4,331,556 discloses a method of combining a crystallizing material with a liquid additive that has properties of forming a metastable solid together with the crystallizing material. When the liquid additive exsolves, the crystalline aggregate is weakened and is easily decomposed into fragments of small size. The liquid additive materials may include monohydric alcohols, diols and triols. The liquid additive material may be included in the liquid to be crystallized, in small amounts, amounts to two percent (2%) to five percent (5%) being typical. The amount and relative metastability of the liquid additive material in the solution contributes to control of the size of the crystals that are ultimately produced when the supercooled fluid crystallizes. A small amount of surface-active material may also be included to modify the characteristics of the metastable solid solution, the exsolution process, and the texture of the exsolved crystal aggregate.

Other materials that can be added to the variable liquid element are gelling agents such as those discussed in US RE035586 or US 5,065,598, which teach the use of one or more materials selected from the group consisting of natural and synthetic gums and gum-like materials such as gelatin; cellulose derivatives such as methyl cellulose and carboxymethylcellulose sodium salt; superabsorbants such as hydrolyzed acrylonitrile grafted starch; synthetic polymer or copolymer which is hydratable and cross-linkable in solution, such as polyacrylates and copolymers of acrylic acid and polyallyl sucrose known as CARBOMER™ from B.F. Goodrich Chemical Company; polyethylene glycol, and any other similar additives known in the art, and mixtures thereof.

### CONTAINER

The trigger mechanism of the present invention is desirably used with, a container that holds the variable liquid element or contains the variable liquid element and another product. The container may comprise any container having one or more non-deformable or generally flexible and/or deformable outer surface such as a wall that allows pressure to be applied through the outer surface to at least a portion of the variable liquid element in order to activate the variable liquid element. The container, for example, may comprise a flexible bottle, a flexible tube, a flexible tub, a sachet, a pouch, a stand-up pouch, a flexible thermoformed container, cartons or the like. The container may be comprised of one or more polymeric materials, metallized structures, laminate structures, lined paperboards, co-extruded films and the like.

If the variable liquid element provides a temperature change, the container may also include some form of insulation, such as insulation layers, that insulates the exterior of the container from the internal variable liquid element and increases the efficiency of the temperature change. The insulation layer may extend over substantially the entire exterior and/or interior surface of the container, may extend over at least a portion of the exterior and/or surface of the container corresponding to about the size of the variable liquid element, may cover a portion of the exterior and/or interior of the outer container that a consumer might touch during the temperature change process. The insulation layer may be a separately attached layer or an integral part of the material forming the container such as a co-extruded or laminated structure. Examples of possible insulating materials include, but are not limited to, foamed polyethylene, silicone rubber, a fibrous cellulose structure, or a dimpled thermoformed film with a rigid thickness to provide air pockets as insulation (lattice with a large amount of void space) and the like.

The container may be formed and/or sealed in any manner known in the art. For example, an outer container formed out of a polymeric film may be sealed via one or more of the following: heat sealing, ultrasonic bonding, radio frequency bonding, adhesive bonding, etc.

If the container is to be used for providing a temperature change for products such as liquids, shampoos, oils, waxes, emollients, lotions, moisturizers, medicaments, pastes, surfactants, gels, adhesives, suspensions, solutions, enzymes, soaps, cosmetics, liniments, beverages, soups, cheeses, flowable particulates and the like, then the container's external surface may also include a useful layer that may be used to perform a function related to the product contained in the package. A container containing a hair treatment product, for example, may include bristles or tines on the outside surface of the outer container to aid in dispensing the product onto hair follicles. A container containing a skin lotion may include an abrasive surface that may be used to exfoliate the user's skin before or after the lotion is applied. The container may also include an absorbent structure that may aid in removing a product from a target surface to which the product has been applied.

In another embodiment, the container may be attached to or housed within a device that may assist in using or dispensing of the product. In the case of a hair treatment product, for example, the container may be housed within a hair-brush or comb that spreads the hair treatment onto hair follicles. Alternatively, a container having a cleaning solution may be housed inside a sponge, brush, wipe, abrasive scrubbing implement, etc. that may be used to scrub a target surface using the product from the container.

The container may further include an optional opening means. For example, the opening means may be, but not limited to, rupturable barriers such as frangible seals, perforations, tear strips, ZIPLOCK™, and the like. Alternatively, the container may be cut open to access the product. The opening may also be sized or designed to prevent the variable liquid element from exiting the container when the product is dispensed if the variable liquid element is not otherwise attached or restrained within the container. For example, the opening may include a tapered dosing channel to prevent the variable liquid element from exiting the container during use. The container may also include a dispensing attachment such as a spout, an applicator tip, spray head or the like in order to aid in product dispensing.

### PERFORATION

The perforation is formed in at least one wall of the container. The perforation can be a puncture, a hole, a slit, a crack, a crevice, or any other similar opening that can be formed in the wall of the container by piercing the wall of the container with hot needles, cold needles, vacuum forming, or high pressure water jet forming or any other method known in the art.

### ACTIVATION MATERIAL

Activation material as used herein means a nucleating particle or seed crystal that, when in contact with the variable liquid element, can cause the desired physical property change. The activation material is brought into contact with the variable liquid element through the perforation contained in the wall of the container. Preferable activation material is a crystal comprising the same material as the variable liquid element. The activation material is keep isolated from the perforation in the wall of the container by the movable barrier means, discussed below.

### MOVABLE BARRIER MEANS

The movable barrier means of the present invention is a device that, when activated by a user, initiates the phase change of the variable liquid element. The movable barrier means remains inactivated until an event, such as pressure or motion, causes the movable barrier means to be activated. The purpose of the movable barrier means is to isolate the variable liquid element from the activation material.

The movable barrier means is located external to the container such that the movable barrier means protects the activation material from external contact as well as provides a removable barrier to the perforation in the wall of the container. For example, the movable barrier means may comprise a folded tab that is activated by a user. The movable barrier means seals, by adhesive or other suitable sealing means, the perforation in the container wall and at the same time isolates the activation material from the variable liquid element. When the user activates the movable barrier means, the movable barrier means opens and brings the activation material into contact with a perforation in the container wall.

The activation material is printed, coated, sprayed, or otherwise be deposited upon a section of the movable barrier means. The movable barrier means is then folded or oriented so that the activation material is not exposed to the perforation in the container wall. Similarly the activation material should not be exposed on the outside surface of the movable barrier means. The movable barrier means may be perforated or scored to make for easy tearing or opening by a user. The movable barrier means is placed over the perforation in the container wall that holds the variable liquid element. The movable barrier means may be activated by pulling or lifting so that the activation material comes into contact with the perforation.

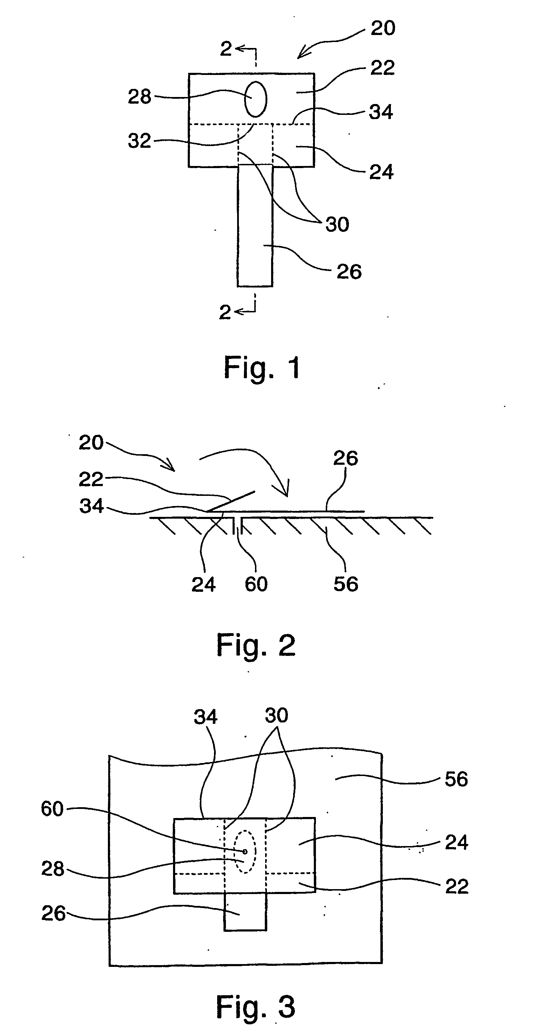

In one preferred embodiment of the movable barrier means 20, shown in Figs. 1, 2 and 3, the movable barrier means 20 is made from one piece of material, such as a plastic laminate or other suitable material, comprising an outer cover 22, an inner cover 24, and a pull-tab 26. Activation material 28 is located on a portion of the outer cover 22. The inner cover 24 contains vertical tear zones 30 which extend from the sides of the pull-tab 26 toward the outer cover 22 through the inner cover 24. The vertical tear zones 30 preferably terminate at a horizontal tear zone 32. The horizontal tear zone preferably comprises a portion of fold line 34. The fold line 34 is located between the outer cover 22 and the inner cover 24.

The outer cover 22 is folded along fold line 34 onto the inner cover 24 and partially onto the pull-tab 26; see Fig. 2. Thus, the activation material 28 is located between the outer cover 22 and the inner cover 24 and there remains isolated until the user wished to initiate activation of the movable barrier means 20. The movable barrier means 20 is preferably attached to a container 56 with a perforation 60 in the container 56.

The movable barrier means 20 is activated when the user pulls the pull-tab 26 with a horizontal motion. The vertical tear zones 30 and horizontal tear zone 32 break away and expose the activation material 28 to the perforation 60 in the container 56.

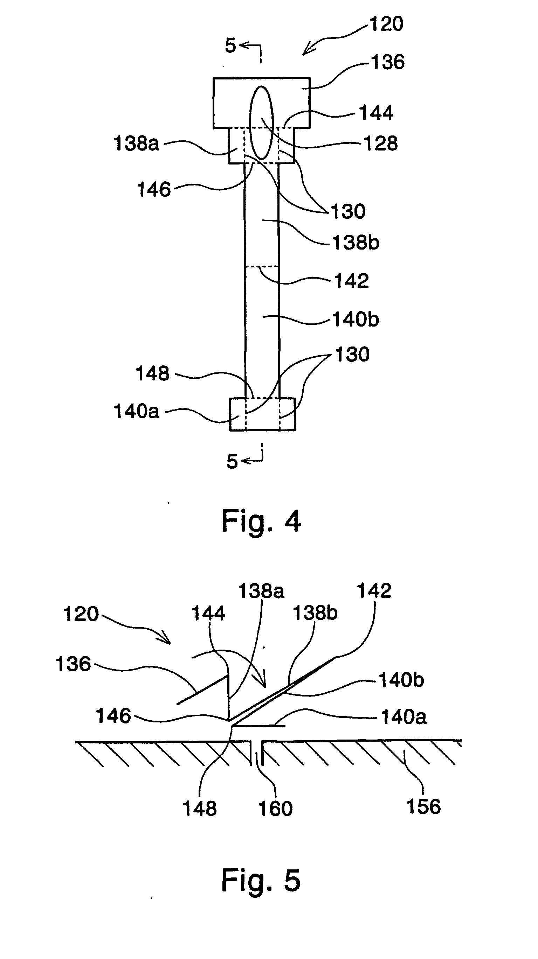

Another preferred embodiment of a movable barrier means 120, shown in Figs. 4, 5 and 6 comprises one piece of material, such as a plastic laminate or other suitable material, which is comprised of three sections. The first section, cover 136, is preferably rectangular in nature, but can be any suitable shape, and is the largest of the three sections.

The cover 136 is separated by fold line 144 from the second section. The second section comprises two sub-sections, crown area 138a and center area 138b, which are orientated in a "T" shape. The crown area 138a and the center area 138b are separated by fold line 146. The crown area 138a contains vertical tear zones 130 extending from the sides of the center area 138b, through the crown area 138a and preferably terminating at fold line 144.

The center area 138b is separated by fold line 142 from the third section. The third section comprises two sub-sections, top area 140a and the middle area 140b, also orientated in a "T" shape structure. The top area 140a and the middle area 140b are separated by fold line 148. The top area 140a contains vertical tear zones 130 extending from the sides of the middle area 140b, through the top area 140a, and preferably terminating at fold line 148.

Activation material 128 preferably is placed on an area encompassing a portion of cover 136 and crown area 138a. Cover 136 is then folded over crown area 138a along fold line 144 so that the activation material 128 is enclosed between cover 136 and crown area 138a. Crown area 138a and center area 138b are folded along fold line 146 in the opposite direction that cover 136 was folded onto crown area 138a, as shown in Fig. 5. Similarly, top area 140a and middle area 140b are folded along fold line 148 in the same direction as fold line 146, as shown in Fig. 5. Center area 138b and middle area 140b are orientated to align by folding fold line 142.

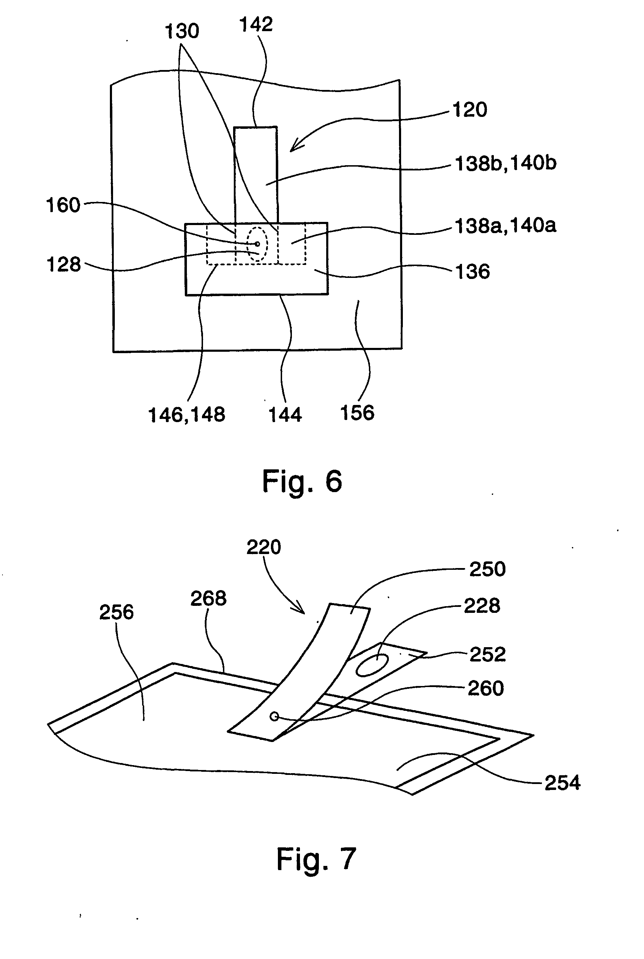

The movable barrier means 120, shown in Fig. 6, is preferably connected to a container 156 having a perforation 160 in the container 156 wall. The movable barrier means 120 is connected to the container 156 over the perforation 160 by attaching the top area 140a to one side of the perforation 160 and the crown area 138a located over the perforation 160. The movable barrier means 120 results with the middle area 140b superpose over the top area 140a, the center area 138b superposed over the middle area 140b, the crown area 138a superposed over the center area 138b and the cover 136 superposed over the crown area 138a; see Fig. 5. The cover 136 is preferably adhesively connected to container 156 around the crown area 138a.

The movable barrier means 120 is activated when the user pulls center area 138b and middle area 140b in a horizontal direction. When the movable barrier means 120 is activated, the vertical tear zones 130 tear away and expose the activation material 128 to the perforation 160 in the container 156.

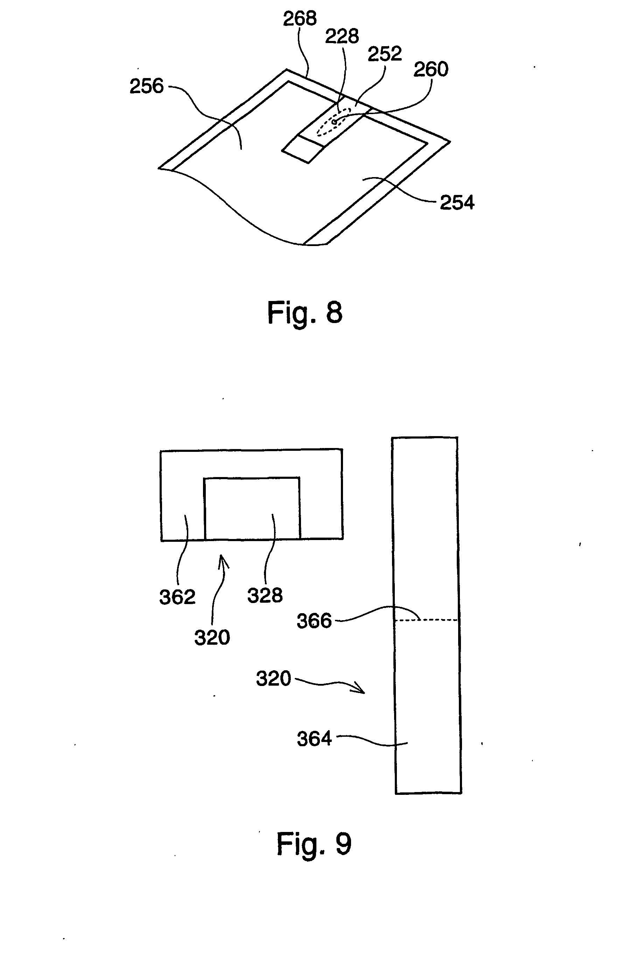

Another preferred embodiment of the movable barrier means 220 is shown in Figs. 7 and 8. The movable barrier means 220 comprises a top tab 250 and a bottom tab 252. Each tab is comprised of a plastic laminate or any other such suitable materials. The activation material 228 is placed on a small area of the bottom tab 252. The bottom tab 252 is connected to the first side 254 of the container such that the small area with the activation material 228 is not in contact with the outer surface of the container. The top tab 250 is adhesively connected to the second side 256 of the container in the same area and in the same orientation as the top tab 250. The movable barrier means 220 is activated when the user removes the top tab 250 from the second side 256 of the container and folds the bottom tab 252 around the edge of the container 258 so that the activation material 228 comes into contact with a perforation 260 in the second side 256 of the container, as shown in Figure 8.

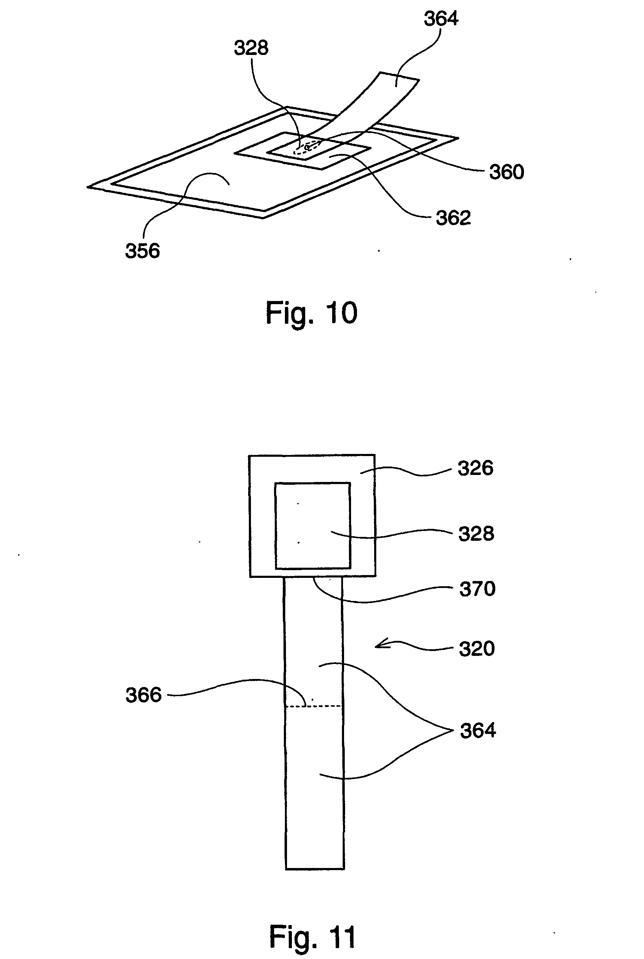

Another preferred embodiment of the movable barrier means 320, shown in Figs. 9 and 10, comprises two pieces of material, cover tape 362 and base tape 364. The tapes are comprised of a plastic laminate or other suitable material. The cover tape 362 is printed, coated, sprayed, or otherwise deposited with activation material 328 on a portion of its surface.

The base tape 364 is preferably folded along fold line 366 to created an area that is easily grasped by the user but still have an area secured to a container 356, but such folding is not required. The base tape 364 is then adhesively placed over a perforation 360 in a container 356. The cover tape 362 is then adhered to the container 356, over a portion of the base tape 364. The movable barrier means 320 may be activated by pulling or lifting the base tape 364 so that it is removed from its position between the perforation 60 in the container 356 and the cover tape 362.

This preferred embodiment alternatively may comprise a single piece of material, such as a plastic laminate or other suitable material, such as shown in Fig. 11 with an additional fold line 370 where the cover tape 362 superposed over the base tape 364.

## Claims

1. A trigger mechanism for initiating a phase change in a variable liquid element comprising:
a) a variable liquid element enclosed in a container having at least one wall;
b) an activation material (28, 128, 228, 328) located external to the wall of the container;
c) at least one perforation (60, 160, 260, 360) in the container wall;
d) a movable barrier means (20, 120, 220, 330) for bringing the activation material (28, 128, 228, 328) into contact with the perforation (60, 160, 260, 360);
wherein the movable barrier means (20, 120, 220, 330) isolates the activation material from the perforation (60, 160, 260, 360) so that the phase change in the variable liquid element is not activated until the movable barrier means (20, 120, 220, 330) is initiated by a user
**characterised in that**
the movable barrier means (20, 120, 220, 330) further comprises a folded tab (26, 250, 252) wherein the activation material is printed, coated, sprayed, or any combination thereof, onto the folded tab; the folded tab (26, 250, 252) is externally connected to the container over the perforation and the folded tab isolates the activation material from the perforation.

2. A trigger mechanism of Claim 1 wherein the container further contains a product, and wherein the phase change in the variable liquid element provides a temperature change to the product.

3. A trigger mechanism of Claim 1 wherein the variable liquid element is a supercooled salt solution.

4. A trigger mechanism of Claim 1 wherein the perforation is a puncture, a hole, a slit, a crack, a crevice, or any combination thereof.

## Patentansprüche

1. Auslösemechanismus zum Initiieren eines Phasenwechsels in einem veränderbaren Flüssigkeitselement, umfassend:
a) ein veränderbares Flüssigkeitselement, das in einem Behälter eingeschlossen ist, der mindestens eine Wand aufweist;
b) ein Aktivierungsmaterial (28,128,228,328), das außerhalb der Wand des Behälters angeordnet ist;
c) mindestens eine Durchbohrung (60,160,260,360) in der Behälterwand;
d) ein bewegliches Sperrmittel (20,120,220,330), um das Aktivierungsmaterial (28,128,228,328) mit der Durchbohrung (60,160,260,360) in Kontakt zu bringen;
wobei das bewegliche Sperrmittel (20,120,220,330) das Aktivierungsmaterial von der Durchbohrung (60,160,260,360) isoliert, so dass der Phasenwechsel in dem veränderbaren Flüssigkeitselement nicht aktiviert wird, bis das bewegliche Sperrmittel (20,120,220,330) von einem Benutzer initiiert wird, **dadurch gekennzeichnet, dass** das bewegliche Sperrmittel (20,120,220,330) ferner einen gefalteten Streifen (26,250,252) umfasst, wobei das Aktivierungsmaterial auf den gefalteten Streifen gedruckt, beschichtet, zerstäubt oder durch eine Kombination davon aufgebracht wird; der gefaltete Streifen (26,250,252) ist von außen mit dem Behälter über der Durchbohrung verbunden und der gefaltete Streifen isoliert das Aktivierungsmaterial von der Durchbohrung.

2. Auslösemechanismus nach Anspruch 1, wobei der Behälter ferner ein Produkt enthält und wobei der Phasenwechsel in dem veränderbaren Flüssigkeitselement für das Produkt eine Temperaturveränderung bereitstellt.

3. Auslösemechanismus nach Anspruch 1, wobei das veränderbare Flüssigkeitselement eine unterkühlte Salzlösung ist.

4. Auslösemechanismus nach Anspruch 1, wobei die Durchbohrung ein Einstich, ein Loch, ein Schlitz, ein Riss eine Spalte oder eine Kombination davon ist.

## Revendications

1. Mécanisme de déclenchement pour lancer un changement de phase dans un élément liquide variable comprenant:
a) un élément liquide variable enfermé dans un récipient ayant au moins une paroi;
b) un matériau d'activation (28, 128, 228, 328) situé à l'extérieur de la paroi du récipient;
c) au moins un orifice (60, 160, 260, 360) dans la paroi du récipient;
d) un moyen de protection amovible (20, 120, 220, 330) pour amener le matériau d'activation (28,128, 228, 328) en contact avec l'orifice (60, 160, 260, 360);
dans lequel le moyen de protection amovible (20, 120, 220, 330) isole le matériau d'activation de l'orifice (60, 160, 260, 360) de telle sorte que le changement de phase dans l'élément liquide variable ne soit pas activé tant que le moyen de protection amovible (20, 120, 220, 330) n'est pas lancé par un utilisateur, **caractérisé en ce que** le moyen de protection amovible (20, 120, 220, 330) comprend en outre une languette pliée (26, 250, 252) dans laquelle le matériau d'activation est imprimé, enduit, vaporisé, ou n'importe quelle combinaison de ces actions, sur la languette pliée; la languette pliée (26, 250, 252) est reliée de manière externe au récipient sur l'orifice et la languette pliée isole le matériau d'activation de l'orifice.

2. Mécanisme de déclenchement selon la revendication 1 dans lequel le récipient contient en outre un produit, et dans lequel le changement de phase dans l'élément liquide variable fournit un changement de température du produit.

3. Mécanisme de déclenchement selon la revendication 1 dans lequel l'élément liquide variable est une solution saline super-refroidie.

4. Mécanisme de déclenchement selon la revendication 1 dans lequel l'orifice est une perforation, un trou, une fente, une entaille, une fissure ou une combinaison de ceux-ci.
